# FASCICULE DE BREVET EUROPEEN

(11) **EP 3 582 824 B1**
(45) Date de publication et mention de la délivrance du brevet: **19.05.2021**
(21) Numéro de dépôt: 18712941.6
(22) Date de dépôt: 16.02.2018
(51) Int. Cl.: A61M 5/142

(54) **DISPOSITIF D'INJECTION AUTOMATIQUE DE PRODUIT FLUIDE**
AUTOMATISCHE INJEKTIONSVORRICHTUNG FÜR FLUIDPRODUKTE
AUTOMATIC FLUID PRODUCT INJECTION DEVICE

(30) Priorité: 20.02.2017 FR 1751321
(43) Date de publication de la demande: 25.12.2019
(73) Titulaire: APTAR France SAS, 27110 Le Neubourg (FR)
(72) Inventeur: BROUET, Guillaume, 76000 Rouen (FR); REEVES, Sam, Cambridge Cambridgeshire CB4 0WS (GB)
(74) Mandataire: CAPRI
(86) Numéro de dépôt international: PCT/FR2018/050375
(87) Numéro de publication internationale: WO 2018/150147

(56) Documents cités:
- EP-A1- 2 179 754
- EP-A2- 2 736 565
- WO-A2-2007/108987
- US-A1- 2007 088 271
- US-A1- 2013 253 430
- US-A1- 2016 144 101

## Description

La présente invention concerne un dispositif d'injection automatique de produit fluide.

Les dispositifs d'injection automatique de produit fluide sont bien connus. Ils comportent notamment les autoinjecteurs, dans lesquels le contenu d'un réservoir, généralement une seringue, est automatiquement injecté au moyen d'un système d'actionnement comprenant généralement un ressort chargé et qui lors du déclenchement va déplacer un piston dans le réservoir pour injecter le produit fluide.

Ces dispositifs de l'art antérieur peuvent présenter des problèmes, notamment lorsque les volumes à distribuer sont importants, lorsque le produit fluide est relativement visqueux ou lorsque plusieurs produits fluides doivent être associés dans un même traitement.

Les documents EP2179754 et US2007088271 décrivent des dispositifs de l'état de la technique.

La présente invention a pour but de fournir un dispositif d'injection automatique qui ne reproduit pas les inconvénients susmentionnés.

La présente invention a aussi pour but de fournir un dispositif d'injection automatique qui permet la distribution automatique de produit fluide, même à des volumes et/ou viscosités importants.

La présente invention a également pour but de fournir un dispositif d'injection automatique de produit fluide qui est simple et peu coûteux à fabriquer et à assembler.

La présente invention, selon les termes de la revendication 1, a donc pour objet un dispositif d'injection automatique de produit fluide comportant un corps de base, destiné à venir au contact d'une zone à injecter, un ensemble support destiné à supporter un mécanisme d'actionnement commandé par des moyens d'alimentation de puissance, au moins un réservoir de produit fluide, contenant chacun un piston d'injection, disposé dans ledit corps de base, un ensemble d'aiguille comportant un actionneur d'insertion, des moyens de déplacement d'aiguille, une aiguille d'amorçage associée à chaque réservoir et destinée à pénétrer dans ledit réservoir avant déplacement dudit piston, et une aiguille d'injection destinée à pénétrer dans la zone à injecter et à injecter le contenu desdits réservoirs dans ladite zone à injecter, ledit dispositif comportant au moins un bouton d'actionnement pour réaliser l'amorçage, l'insertion de l'aiguille d'injection dans la zone à injecter, l'administration du produit fluide puis la rétractation de l'aiguille d'injection.

Avantageusement, ledit corps de base comporte un autocollant pour se fixer sur la zone à injecter.

Avantageusement, ledit corps de base comporte au moins deux réservoirs, notamment trois.

Avantageusement, les produits fluides contenus dans lesdits réservoirs sont distribués simultanément, en étant mélangé en amont de ladite aiguille d'injection.

Selon une variante avantageuse, les produits fluides contenus dans lesdits réservoirs sont distribués successivement.

Avantageusement, un bouton d'actionnement respectif est associé à chaque réservoir.

Selon une variante avantageuse, le dispositif comporte un seul bouton d'actionnement.

Avantageusement, le mécanisme d'actionnement comprend pour chaque réservoir un cylindre contenant un fluide d'actionnement, tel qu'une solution saline, dans lequel peut coulisser un plongeur respectif commandé par un mécanisme d'entrainement.

Avantageusement, ledit mécanisme d'entrainement comprend un moteur électrique associé à un engrenage à vis sans fin ou à un système d'engrenages du type horloger.

Avantageusement, ledit actionneur d'insertion, avantageusement un actionneur électromécanique, tel qu'un solénoïde, est actionné après amorçage d'au moins un desdits réservoirs par lesdites aiguilles d'amorçage, ledit actionneur d'insertion étant relié à ladite aiguille d'injection au moyen d'un ou plusieurs bras pivotant(s) pour faire pénétrer ladite aiguille d'injection dans ladite zone à injecter.

Avantageusement, ledit actionneur d'insertion est actionné après distribution des contenus de tous les réservoirs pour rétracter ladite aiguille d'injection de ladite zone à injecter.

Avantageusement, chaque réservoir contient entre 1 et 10 ml de produit fluide, avantageusement environ 3 ml.

Avantageusement, lesdits moyens d'alimentation de puissance comportent une pile et/ou un accumulateur d'énergie électrique.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective éclatée d'un dispositif d'injection automatique selon un mode de réalisation avantageux,
- la figure 2 est une vue schématique en perspective de l'intérieur du dispositif de la figure 1,
- les figures 3 et 4 sont des vues schématiques de dessus, de deux variantes de réalisation différentes des moyens d'actionnement du dispositif des figures 1 et 2,
- les figures 5 à 7 sont des vues schématiques de côté de la séquence d'actionnement du dispositif des figures 1 et 2,
- la figure 8 est une vue schématique partielle en perspective de l'ensemble d'aiguille, avant piquage,
- les figures 9 et 10 sont des vues schématiques de côté de l'ensemble d'aiguille, respectivement avant et après piquage, et
- les figures 11 à 13 sont des vues schématiques en perspectives de diverses variantes de réalisation.

L'invention concerne un dispositif d'injection automatique particulièrement adapté à distribuer des volumes relativement importants de produit fluide, typiquement de l'ordre de quelques millilitres, typiquement de 1 à 10 ml, par exemple 3 ml. Le dispositif de l'invention est aussi adapté à distribuer des produits fluides relativement visqueux.

Le dispositif est de préférence jetable et peut fonctionner avec les étapes d'utilisation suivantes:
1) l'utilisateur retire l'emballage et fixe le dispositif sur une zone à injecter, par exemple au moyen d'un autocollant prévu à cet effet;
2) l'utilisateur appuie sur un bouton d'actionnement 200 pour actionner le dispositif, ce qui provoque notamment l'amorçage du dispositif, l'insertion de l'aiguille d'injection dans la zone à injecter, l'administration du produit fluide puis la rétractation de l'aiguille d'injection;
3) l'utilisateur est alerté de l'achèvement du processus, il détache l'appareil du corps et le jette.

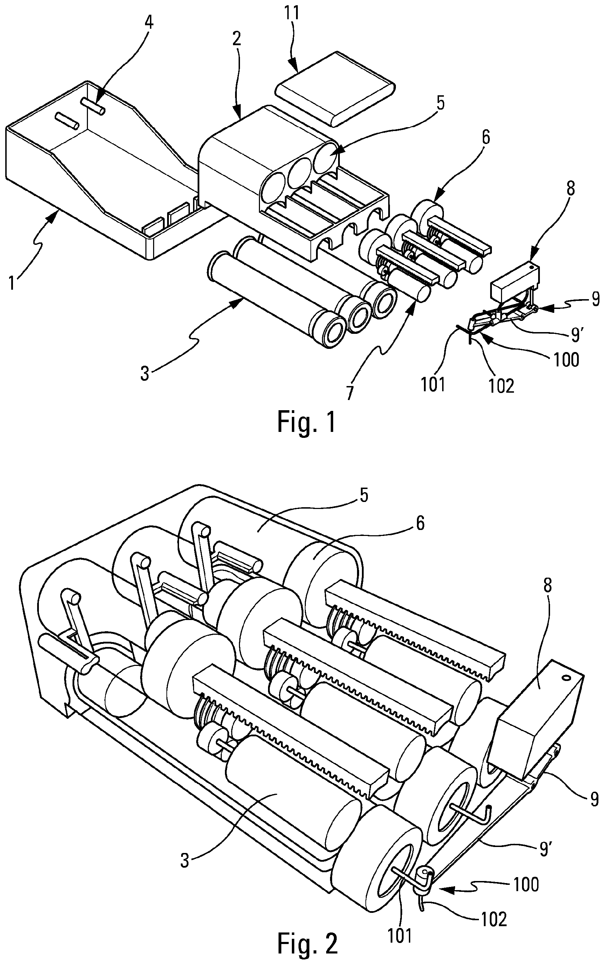

Sur les figures 1 et 2, il est représenté un dispositif d'injection automatique selon un premier mode de réalisation avantageux.

Le dispositif comprend un corps de base 1, destinée à venir au contact de la zone à injecter, un ensemble support 2, destiné à supporter un mécanisme d'actionnement 5, 6, 7, un ou plusieurs réservoirs 3 de produit fluide contenant chacun un piston d'injection 35, disposé(s) dans ledit corps de base 1, un ensemble d'aiguille 100 comportant un actionneur d'insertion 8, des moyens de déplacement d'aiguille 9, 9', une ou plusieurs aiguille(s) d'amorçage 101 destinée(s) à pénétrer dans le ou les réservoirs 3, et une aiguille d'injection 102 destinée à pénétrer dans la zone à injecter, et des moyens d'alimentation de puissance 11.

Dans l'exemple représenté, l'alimentation 11 est une pile ou un accumulateur d'énergie électrique.

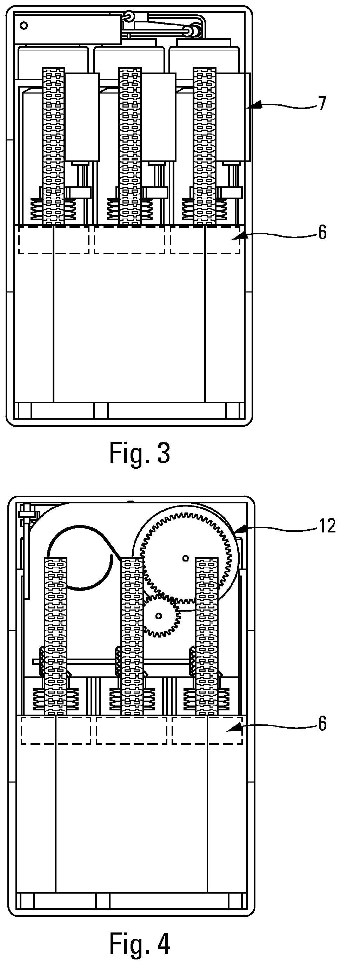

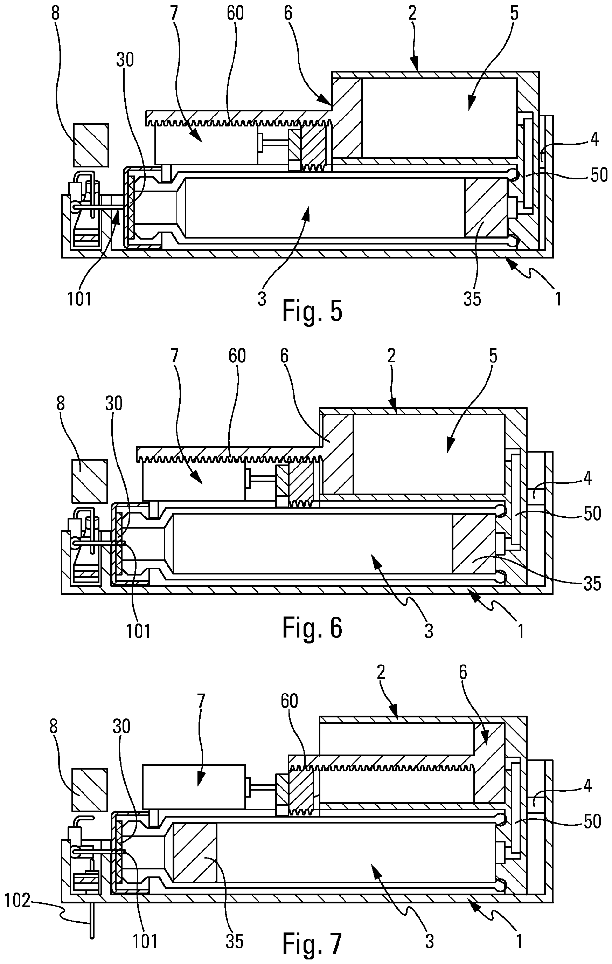

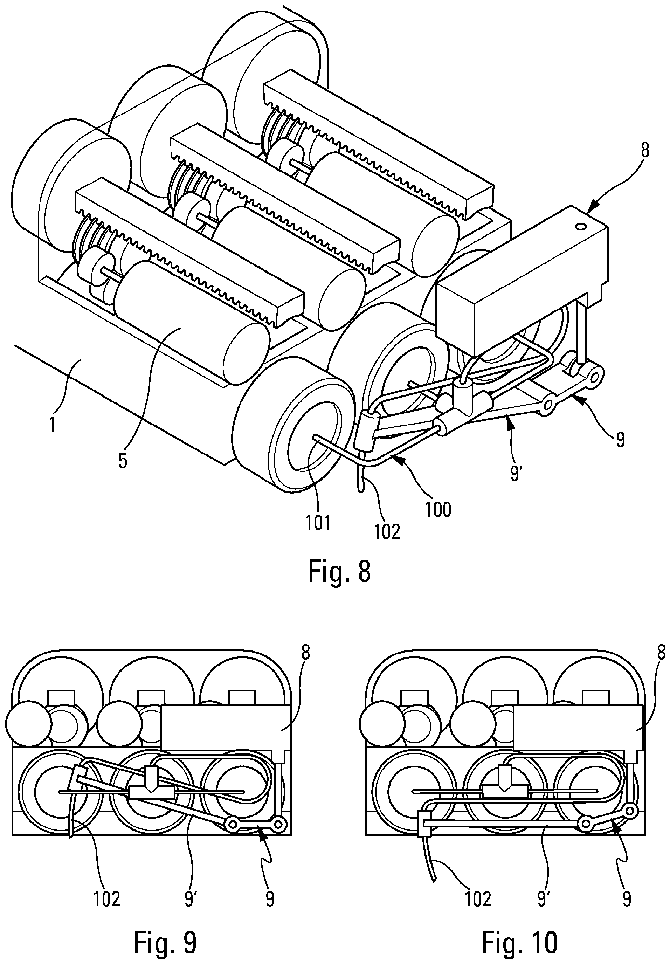

Le mécanisme d'actionnement dans cet exemple est du type hydraulique, avec au moins un cylindre 5 contenant un fluide d'actionnement, tel qu'une solution saline, dans lequel peut coulisser un plongeur 6 respectif commandé par un mécanisme d'entrainement 7. Après actionnement du bouton d'actionnement 200, la source d'alimentation 11 entraine le plongeur 6 dans un mouvement linéaire afin de comprimer un fluide d'actionnement contenu dans le cylindre 5. Le plongeur 6 peut être déplacé par un certain nombre de procédés connus, comprenant un moteur électrique associé à un engrenage à vis sans fin 7, comme représenté sur les figures 1 à 3 et 5 à 8, ou à un système d'engrenages du type horloger 12, comme représenté sur la figure 4. Des mécanismes d'actionnement autres qu'hydrauliques, par exemple mécaniques, pneumatiques, thermiques ou chimiques, sont utilisables en variante.

Avantageusement, le ou les réservoirs 3 sont obturés avant actionnement par une membrane 30 formant septum, destinée à être percée par une aiguille d'amorçage 101 lors de l'actionnement. A cet effet, le dispositif comporte un ou plusieurs piston(s) d'amorçage 4 destiné(s) à déplacer ledit ensemble support 2 par rapport audit corps de base 1 au début de l'actionnement, pour réaliser l'amorçage en perçant la ou les membrane(s) 30.

Avantageusement, les moyens de déplacement d'aiguille 9 comportent un ou plusieurs bras articulé(s) 9, 9' supportant l'aiguille d'injection 102 commandé(s) par l'actionneur d'insertion 8. Cet actionneur d'insertion 8 peut être un actionneur électromécanique, tel qu'un solénoïde.

Dans le dispositif décrit ci-dessus, les opérations de perçage du septum et de distribution du produit fluide sont toutes deux actionnées par le même cylindre d'actionnement 5. Le cylindre d'actionnement 5, le plongeur 6, le mécanisme d'entrainement 7 et le réservoir 3 sont tous solidaires de l'ensemble support 2. Celui-ci peut coulisser par rapport au corps de base 1 qui est fixée au patient. L'ensemble d'aiguille 100 est fixe par rapport au corps de base 1.

Lors de l'actionnement, le mécanisme d'entrainement 7 du plongeur 6 amène le plongeur 6 à s'étendre, ce qui déplace le fluide hydraulique. Ceci provoque l'extension du ou des piston(s) d'amorçage 4 qui déplace l'ensemble support 2 complet, y compris les réservoirs 3, par rapport au corps de base 1. Les réservoirs 3 sont forcés contre l'ensemble d'aiguille 100, perçant les septums au moyen des aiguilles d'amorçage 101. Le dispositif est maintenant amorcé.

Après que le dispositif a été amorcé, l'aiguille d'injection 102 est insérée dans la zone à injecter du patient au moyen d'un actionneur d'insertion 8, avantageusement un actionneur électromécanique, tel qu'un solénoïde. Cet actionneur 8 est relié à l'aiguille d'injection 102 au moyen d'un ou plusieurs bras pivotant(s) 9, 9'.

Si plusieurs cartouches sont utilisées, comme représenté dans l'exemple des figures 1 et 2, les aiguilles d'amorçage 101 de tous les réservoirs 3 sont couplées à une seule aiguille d'injection 102.

Au repos, l'actionneur 8 est en position déployée. L'ensemble aiguille 100 et les bras pivotants 9, 9' sont en position rétractée.

L'aiguille d'injection 102 est déployée après que le dispositif a atteint l'état amorcé. Ceci peut être déterminé par exemple au moyen d'un contrôleur mécanique et/ou logiciel, par exemple après que le ou les pistons d'amorçage 4 sont complètement étendus. Lorsqu'il est déployé, l'actionneur 8 se rétracte ce qui amène les bras pivotants 9, 9' à déplacer l'aiguille d'injection 102 dans la zone à injecter. L'aiguille d'injection 102 est avantageusement courbée de telle sorte qu'elle peut fléchir pour permettre le mouvement de la pointe d'aiguille. Cette forme courbée est aussi avantageuse pour s'adapter au mouvement de pivotement des bras pivotants 9, 9'.

Après insertion de l'aiguille d'injection, le mécanisme d'entrainement 7 continue à étendre le plongeur 6. Cela déplace plus de fluide hydraulique à travers un canal 50 de l'ensemble support 2, à l'arrière du réservoir 3, ce qui entraîne le piston 35 du réservoir vers l'avant et délivre le produit fluide. La force requise pour cette opération est supérieure à celle requise pour entraîner les pistons d'amorçage 4 pour s'assurer que l'amorçage se produit en premier.

Une fois que suffisamment de produit fluide a été injecté, le processus est terminé en arrêtant le fonctionnement du mécanisme d'entrainement 7.

Lorsque la distribution du produit fluide est terminée, l'actionneur d'insertion 8 rétracte l'aiguille dans le dispositif. La fin de la distribution du produit fluide peut être identifiée par un contrôle mécanique et/ou logiciel, par exemple après que le mécanisme d'entrainement 7 est complètement étendu.

Le mode de réalisation décrit fournit notamment les avantages suivants:
- perçage du septum et délivrance de médicament par le même mécanisme, minimisant le nombre de composants;
- contrôle précis du débit de produit fluide;
- forme compacte;
- la pointe de l'aiguille d'injection est incurvée pour minimiser l'inconfort du patient pendant le processus d'injection;
- le cheminement du fluide est simple, ce qui minimise le risque de contamination.

Le mode de réalisation illustré sur les figures 1 et 2 montre un dispositif adapté à comporter un, deux ou trois réservoirs 3. On peut prévoir l'utilisation de masques sur l'ensemble d'aiguille qui, lorsque le dispositif est amorcé, sont perforés/pénétrés par la présence du réservoir. Lorsque le réservoir n'est pas présent, le masque agit pour sceller la branche respective de l'ensemble d'aiguille, empêchant la fuite de médicament pendant la distribution.

Le dispositif représenté sur les figures 1 et 2 comporte trois mécanismes d'entrainement 7 agissant sur trois réservoirs 3. Les trois mécanismes d'entrainement 7 peuvent être actionnés simultanément, au moyen d'un seul bouton d'actionnement 200, pour distribuer simultanément les contenus des trois réservoirs, qui sont alors mélangés en amont de l'aiguille d'injection 102. En variante, les trois mécanismes d'entrainement 7 peuvent être actionnés successivement, pour distribuer successivement les contenus des trois réservoirs. Ces actionnements successifs peuvent être déclenchés au moyen de trois boutons d'actionnement 200 distincts, mais on pourrait aussi prévoir un seul bouton d'actionnement 200 qui déclenche automatiquement la séquence de distribution. Un mélange de ces deux variantes est aussi possible, par exemple une distribution en deux temps, avec d'une part la distribution du contenu d'un réservoir et d'autre part la distribution simultanée du mélange des deux autres réservoirs.

Le mode de réalisation décrit fournit notamment les avantages suivants:
- la vitesse de distribution du produit fluide peut être ajustée pour optimiser les traitements individuels et peut également varier dans le temps;
- les mécanismes d'entrainement et les réservoirs multiples permettent l'utilisation d'une combinaison de médicaments qui peuvent être distribués à des vitesses différentes et à des moments différents.

L'utilisation d'un dispositif à un ou plusieurs réservoirs permet de fournir notamment les avantages suivants:
- dispositif unique pour plusieurs types de produits fluides, qui peut nécessiter la distribution de volumes différents;
- possibilité de distribuer des cocktails ou mélange de plusieurs produits fluides;
- possibilités d'associer des agents réducteurs de douleur (anesthésiques, neutralisateur d'acidité, etc.) au médicament à injecter;
- possibilité d'avoir des fréquences de traitement de médicaments différents; par exemple une première séquence S1 avec la prise de plusieurs médicaments différents, suivie d'une seconde séquence S2 avec la prise d'un seul médicament, etc.;
- possibilité de standardiser le dispositif d'injection pour plusieurs types de traitement;
- diminution du coût de développement des dispositifs;
- possibilité d'ajustements dans la formulation du produit fluide.
- diverses formulations de produits fluides peuvent être logées dans un seul dispositif;
- réduction du nombre d'injections.

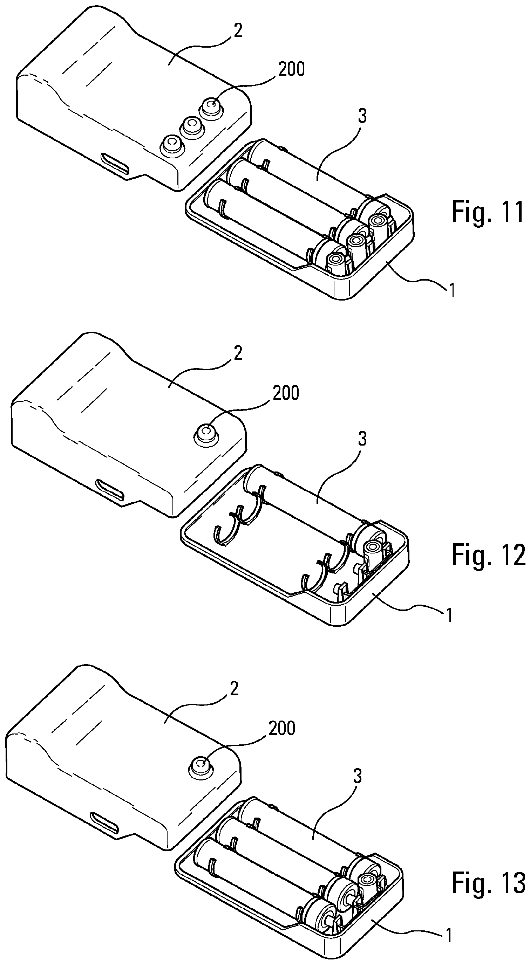

D'autres configurations possibles, par exemple:
- un seul mécanisme d'entrainement 7 actionnant un seul réservoir (figure 12) ;
- un seul mécanisme d'entrainement 7 actionnant simultanément plusieurs réservoirs, en particulier trois (figure 13).

On peut aussi envisager l'utilisation d'ensembles d'aiguilles multiples dans un même dispositif avec un contrôle indépendant à la fois de l'insertion d'aiguille et de l'injection du produit fluide. Ceci permettrait:
- une distribution séquentielle du produit fluide pour un débit réduit;
- la réduction de la taille de l'aiguille d'injection pour augmenter le débit du produit fluide et réduire la douleur du patient;
- l'optimisation du diamètre de l'aiguille d'injection pour une formulation de médicament donnée dans le cas où divers médicaments sont contenus dans le même dispositif.

L'utilisation d'injections simultanées ou séquentielles peut être appliquée dans un système à plusieurs cartouches pour:
- réduire le débit de produit fluide et soulager la douleur du patient;
- permettre une amélioration de l'efficacité de certaines préparations de cocktail de médicaments.

La présente invention a été décrite en référence à plusieurs modes et variantes de réalisation avantageux, mais il est entendu qu'un homme du métier peut y apporter toutes modifications, sans sortir du cadre de la présente invention tel que défini par les revendications annexées.

## Revendications

1. Dispositif d'injection automatique de produit fluide, le dispositif comportant un corps de base (1), destiné à venir au contact d'une zone à injecter, un ensemble support (2) monté coulissant par rapport au corps de base (1) et supportant un mécanisme d'actionnement (5, 6, 7) commandé par des moyens d'alimentation de puissance (11), au moins un piston d'amorçage (4) assurant le déplacement dudit ensemble support (2) par rapport audit corps de base (1), au moins un réservoir (3) de produit fluide, contenant chacun un piston d'injection (35), disposé dans ledit corps de base (1), un ensemble d'aiguille (100) fixe par rapport au corps de base (1) et comportant un actionneur d'insertion (8), des moyens de déplacement d'aiguille (9, 9'), une aiguille d'amorçage (101) associée à chaque réservoir (3) et pénétrant dans ledit réservoir (3) avant déplacement dudit piston (35), et une aiguille d'injection (102) destinée à pénétrer dans la zone à injecter et à injecter le contenu desdits réservoirs (3) dans ladite zone à injecter, ledit dispositif comportant au moins un bouton d'actionnement (200) déclenchant l'amorçage, l'insertion de l'aiguille d'injection dans la zone à injecter, l'administration du produit fluide puis la rétractation de l'aiguille d'injection.

2. Dispositif selon la revendication 1, dans lequel ledit corps de base (1) comporte un autocollant pour se fixer sur la zone à injecter.

3. Dispositif selon la revendication 1 ou 2, dans lequel ledit corps de base comporte au moins deux réservoirs (3), notamment trois.

4. Dispositif selon la revendication 3, comportant des moyens pour distribuer simultanément les produits fluides contenus dans lesdits réservoirs (3).

5. Dispositif selon la revendication 3, comportant des moyens pour distribuer successivement les produits fluides contenus dans lesdits réservoirs (3).

6. Dispositif selon l'une quelconque des revendications précédentes, dans lequel un bouton d'actionnement (200) respectif est associé à chaque réservoir (3).

7. Dispositif selon l'une quelconque des revendications 1 à 5, comportant un seul bouton d'actionnement (200).

8. Dispositif selon l'une quelconque des revendications précédentes, dans lequel le mécanisme d'actionnement comprend pour chaque réservoir (3) un cylindre (5) contenant un fluide d'actionnement, tel qu'une solution saline, dans lequel peut coulisser un plongeur (6) respectif commandé par un mécanisme d'entrainement (7, 12).

9. Dispositif selon la revendication 8, dans lequel ledit mécanisme d'entrainement comprend un moteur électrique associé à un engrenage à vis sans fin (7) ou à un système d'engrenages du type horloger (12).

10. Dispositif selon l'une quelconque des revendications précédentes, comportant des moyens pour actionner ledit actionneur d'insertion (8), avantageusement un actionneur électromécanique, tel qu'un solénoïde, après amorçage d'au moins un desdits réservoirs (3) par lesdites aiguilles d'amorçage (101), ledit actionneur d'insertion (8) étant relié à ladite aiguille d'injection (102) au moyen d'un ou plusieurs bras pivotant(s) (9, 9') pour faire pénétrer ladite aiguille d'injection (102) dans ladite zone à injecter.

11. Dispositif selon la revendication 10, comportant des moyens pour actionner ledit actionneur d'insertion (8) après distribution des contenus de tous les réservoirs (3) pour rétracter ladite aiguille d'injection (102) de ladite zone à injecter.

12. Dispositif selon l'une quelconque des revendications précédentes, dans lequel chaque réservoir contient entre 1 et 10 ml de produit fluide, avantageusement environ 3 ml.

13. Dispositif selon l'une quelconque des revendications précédentes, dans lequel lesdits moyens d'alimentation de puissance (11) comportent une pile et/ou un accumulateur d'énergie électrique.

## Patentansprüche

1. Automatische Injektionsvorrichtung für Fluidprodukte, umfassend einen Grundkörper (1), der dazu bestimmt ist, mit einem Injektionsbereich in Kontakt zu kommen, eine Trägereinheit (2), die in Bezug auf den Grundkörper (1) verschiebbar gelagert ist und einen durch Stromversorgungsmittel (11) gesteuerten Betätigungsmechanismus (5, 6, 7) trägt, mindestens einen Anstechkolben (4), der die Verschiebung der Trägereinheit (2) in Bezug auf den Grundkörper (1) erzeugt, mindestens einen in dem Grundkörper (1) angeordneten Vorratsbehälter (3) für ein Fluidprodukt, der jeweils einen Injektionskolben (35) enthält, eine in Bezug auf den Grundkörper (1) feststehend angeordnete Nadelanordnung (100) umfassend einen Einstechaktuator (8), Nadelverschiebemittel (9, 9'), eine Anstechnadel (101), die jedem Vorratsbehälter (3) zugeordnet ist und vor der Verschiebung des Kolbens (35) in den Vorratsbehälter (3) eindringt, und eine Injektionsnadel (102), die dazu bestimmt ist, in den Injektionsbereich einzudringen und den Inhalt des Vorratsbehälters (3) in den Injektionsbereich zu injizieren, wobei die Vorrichtung mindestens einen Betätigungsknopf (200) zur Einleitung des Anstechens, des Einstechens der Injektionsnadel in den Injektionsbereich, der Verabreichung des Fluidprodukts und des anschließenden Zurückziehens der Injektionsnadel umfasst.

2. Vorrichtung nach Anspruch 1, bei der der Grundkörper (1) einen Aufkleber zu dessen Fixierung auf dem Injektionsbereich aufweist.

3. Vorrichtung nach Anspruch 1 oder 2, bei der der Grundkörper mindestens zwei, insbesondere drei Vorratsbehälter (3) aufweist.

4. Vorrichtung nach Anspruch 3, umfassend Mittel zur gleichzeitigen Abgabe der in den Vorratsbehältern (3) enthaltenen Fluidprodukte.

5. Vorrichtung nach Anspruch 3, umfassend Mittel zur sequentiellen Abgabe der in den Vorratsbehältern (3) enthaltenen Fluidprodukte.

6. Vorrichtung nach einem der vorhergehenden Ansprüche, bei der jedem Vorratsbehälter (3) eine jeweilige Betätigungstaste (200) zugeordnet ist.

7. Vorrichtung nach einem der Ansprüche 1 bis 5, die nur eine Betätigungstaste (200) umfasst.

8. Vorrichtung nach einem der vorhergehenden Ansprüche, bei der der Betätigungsmechanismus für jeden Vorratsbehälter (3) einen Zylinder (5) umfasst, der ein Betätigungsströmungsmittel, beispielsweise eine Kochsalzlösung, enthält, in dem jeweils ein von einem Antriebsmechanismus (7, 12) gesteuerter Kolben (6) verschiebbar angeordnet ist.

9. Vorrichtung nach Anspruch 8, bei der der Antriebsmechanismus einen Elektromotor umfasst, der mit einem Schneckengetriebe (7) oder einem uhrwerkartigen Getriebesystem (12) verbunden ist.

10. Vorrichtung nach einem der vorhergehenden Ansprüche, umfassend Mittel zum Betätigen des Einstechaktuators (8), vorteilhafterweise einen elektromechanischen Aktuator wie beispielsweise ein Solenoid, nach dem Anstechen mindestens eines der Vorratsbehälter (3) durch die Anstechnadeln (101), wobei der Einstechaktuator (8) über einen oder mehrere Schwenkarm(e) (9, 9') mit der Injektionsnadel (102) verbunden ist, um das Einstechen der Injektionsnadel (102) in den Injektionsbereich zu bewirken.

11. Vorrichtung nach Anspruch 10, umfassend Mittel zum Betätigen des Einstechaktuators (8) nach Abgabe des Inhalts aller Vorratsbehälter (3), um die Injektionsnadel (102) wieder aus dem Injektionsbereich herauszuziehen.

12. Vorrichtung nach einem der vorhergehenden Ansprüche, bei der jeder Vorratsbehälter zwischen 1 ml und 10 ml Fluidprodukt enthält, vorzugsweise etwa 3 ml.

13. Vorrichtung nach einem der vorhergehenden Ansprüche, bei der die Stromversorgungsmittel (11) eine Batterie und/oder einen elektrischen Energiespeicher umfassen.

## Claims

1. An automatic fluid injection device, the device comprising a base body (1), for coming into contact with an injection zone, a support assembly (2) slidably mounted relative to the base body (1) and supporting an actuator mechanism (5, 6, 7) that is controlled by power supply means (11), at least a priming piston (4) ensuring the moving of said support assembly (2) relative to said base body (1), at least one fluid reservoir (3), each containing an injection piston (35), arranged in said base body (1), a needle assembly (100) fixed relative to the base body (1) and comprising an insertion actuator (8), needle movement means (9, 9'), a priming needle (101) for associating with each reservoir (3) and penetrating into said reservoir (3) before moving said piston (35), and an injection needle (102) for penetrating into the injection zone and for injecting the content(s) of said reservoir(s) (3) into said injection zone, said device including at least one actuator button (200) triggering the priming, the insertion of the injection needle into the injection zone, the administration of fluid and then the retraction of the injection needle.

2. A device according to claim 1, wherein said base body (1) includes a sticker for fastening onto the injection zone.

3. A device according to claim 1 or claim 2, wherein said base body includes at least two reservoirs (3), in particular three reservoirs.

4. A device according to claim 3, including means for dispensing the fluids contained in said reservoirs (3) simultaneously.

5. A device according to claim 3, including means for dispensing the fluids contained in said reservoirs (3) successively.

6. A device according to any preceding claim, wherein a respective actuator button (200) is associated with each reservoir (3).

7. A device according to any one of claims 1 to 5, including a single actuator button (200).

8. A device according to any preceding claim, wherein, for each reservoir (3) the actuator mechanism includes a cylinder (5) containing a driving fluid, such as a saline solution, in which cylinder there may slide a respective plunger (6) that is controlled by a drive mechanism (7, 12).

9. A device according to claim 8, wherein said drive mechanism comprises an electric motor associated with a worm gear (7) or with a clockmaker-type gear train (12).

10. A device according to any preceding claim, including means for actuating said insertion actuator (8), advantageously an electromechanical actuator, such as a solenoid, after at least one of said reservoirs (3) has been primed by said priming needles (101), said insertion actuator (8) being connected to said injection needle (102) by means of one or more pivot arms (9, 9') so as to cause said injection needle (102) to penetrate into said injection zone.

11. A device according to claim 10, including means for actuating said insertion actuator (8) after the contents of all of the reservoirs (3) have been dispensed so as to retract said injection needle (102) from said injection zone.

12. A device according to any preceding claim, wherein each reservoir has a fluid content in the range 1 mL to 10 mL, advantageously about 3 mL.

13. A device according to any preceding claim, wherein said power supply means (11) comprise an optionally rechargeable battery.
